# EUROPEAN PATENT SPECIFICATION

(11) **EP 0 453 316 B1**
(45) Date of publication and mention of the grant of the patent: **23.08.1995**
(21) Application number: 91303553.1
(22) Date of filing: 19.04.1991
(51) Int. Cl.: B01J 13/02, C12N 11/02

(54) **Process for preparation of microcapsules**
Verfahren zur Herstellung von Mikrokapseln
Procédé de préparation de microcapsules

(30) Priority: 20.04.1990 JP 104432/90
(43) Date of publication of application: 23.10.1991
(73) Proprietor: MITSUBISHI PAPER MILLS, LTD., Chiyoda-ku Tokyo (JP); KIRIN BREWERY COMPANY LTD., Shibuya-ku Tokyo-to (JP)
(72) Inventor: Inoue, Chiaki, Tsukuba-shi (JP); Ishiguro, Mamoru, Tsuchiura-shi (JP); Ishiwaki, Naomu, Takasaki-shi (JP); Yamada, Katsuhiko, Urawa-shi (JP)
(74) Representative: Clifford, Frederick Alan

(56) References cited:
- DD-A- 247 570
- DE-A- 3 530 562
- DE-A- 3 821 619
- GB-A- 2 162 147
- US-A- 4 001 480

## Description

### BACKGROUND OF THE INVENTION

### Field of the Invention

The present invention relates to a process for the preparation of microcapsules which comprise yeast cells as the microcapsule wall.

### Related Art

Microcapsules are fine particles 1 »m to several hundred »m in size which contain a liquid, solid, or gas enclosed therein and a thin film uniformly covering the surrounding thereof. More specifically, microcapsules are being produced commercially which contain colorless or colored dyes, pharmaceuticals, agricultural chemicals, perfumes, feed materials, food materials, etc. enclosed therein.

Microcapsules make it possible to confine the characteristic functions of a substance within the capsules by forming thin film outside the substance having said characteristics as a wall and to take out the enclosed substance when necessary by breaking the film.

Important processes for the preparation of microcapsules so far known include
(1) the coacervation method using gelatin (USP Nos. 2,800,457 and 2,800,458, etc.),
(2) the in situ method wherein film is formed from the outer phase (aqueous phase) (JP-B-Nos. 36-9,168 and 47-23,165, JP-A-Nos. 48-57,892, 51-9,079 and 54-25,277, etc.), and
(3) the interfacial polymerization method which utilizes a film forming reaction between the inner phase and the outer phase.

In addition, USP No. 4,001,480 discloses a process which utilizes cells of a microorganism belonging to Eumycetes having a lipid content of 40-60% by weight as microcapsules by dissolving a fat or lipid soluble substance into the cellular lipid and thus confining it in the cell.

Furthermore, JP-A-No. 58-107,189 discloses microorganism capsules obtained by incorporating a liquid selected from organic substances for extending cellular lipid (e.g., aliphatic alcohols, esters, aromatic hydrocarbons, and hydrogenated aromatic hydrocarbons) into the cells of a grown microorganism having a lipid content of 10% by weight or more harvested from the culture medium (for example, lipid-accumulating yeast, brewer's yeast, etc.), to extend the lipid content of the microorganism, and then dissolving a liquid soluble in said organic substance for extending lipid which is to be a core substance, in the resulting lipid-organic substance mixture and thus confining it in the microorganism cells to form microcapsules.

The above-mentioned method of encapsulation using synthetic resins can provide microcapsules having a dense film excellent in the ability of protecting the encapsulated substances and is widely used commercially, but it is also true that the method actually involves various problems with respect to the aspect of production. That is, the coacervation method of (1) has problems of complicated operations necessary for adjusting the pH, temperature and time of the reaction and of a long time required for the encapsulation step. The in situ method of (2) and the interfacial polymerization method of (3) have a disadvantage in that they are not suitable to the encapsulation of unsatable substances or readily thermally denaturable substances because highly reactive film base materials are subjected to a reaction at relatively high temperature in these method.

The microencapsulation methods utilizing microorganism comprise using a naturally occurring substance, more particularly a part of a being, as one of the materials of the microcapsule, and the mechanism of encapsulation is utterly different in nature from those of the previous methods. However, the methods, as can be seen from the working examples shown in the patent specifications, have disadvantages in that the amount of a hydrophobic liquid which can be enclosed with the initially added yeast cells (i.e., wall material) is relatively samll as compared with those attainable by the methods known previously and commercially used at present, and further a long time is required for encapsulation when intake of a large amount of the hydrophobic liquid is intended.

### Disclosure of the Invention

The object of the present invention is to provide a process for the preparation of microcapsules using yeast cells which can make a large amount of a hydrophobic liquid enclosed in the microcapsules.

In order to solve the above-mentioned problems involved in the encapsulation methods using microorganisms, the present inventors have made extensive study on the method for enclosing a large amount of hydrophobic liquid in yeast cells and resultantly found that the problems can be solved by the following technique.

That is, it has been found that in the process for the preparation of microcapsules comprising yeast cells and a hydrophobic liquid enclosed therein, the amount of the enclosed hydrophobic liquid can be increased by using yeast cells from which intracellular components, more specifically cytoplasma components consisting of various proteins, amino acids, nucleic acids, succharides, etc., have been dissolved out previously.

According to the process of the present invention, there is provided a process for producing microcapsules from yeast cells comprising eluting intracellular components of yeast cells in suspension to outside of the yeast cells; and mixing the suspension of the yeast cells, from which intracellular components have been eluted, with a hydrophobic liquid thereby to obtain microcapsules containing the hydrophobic liquid; characterised in that the yeast cells have a low lipid-content of less than 10% by weight and are subjected to elution treatment at a temperature of 30 to 100°C for a period of time of not less than 1 hour in the absence or presence of an elution promoting agent, so that the intracellular components are eluted at a rate of 10 to 80% by weight and the yeast is a member selected from the group consisting of Saccharomyces cerevisiae, Saccharomyces rouxii, Saccharomyces carlsbergensis, Candida utilis, Candida tropicalis, Candida lipolytica and Candida flaveri.

In the process of the present invention, it has become possible to encapsulate a larger amount of hydrophobic liquids more rapidly than in the known encapsulation methods.

The present invention is to be distinguished from that described in DD-A 247570. In that publication treatment of a yeast suspension is envisaged of such a nature that inner cell components are extracted to leave a porous cellular material. In contrast to this, the present invention proposes to produce a cellular material which can be loaded with a hydrophobic solution of perfume or ink (for example) retainable until mechanical rupture of the cell wall. This is achieved by selection of starting material and control of the conditions as set out above and by the optional use of an elution-promoting agent or an enzyme.

According to the process of the present invention, further, protein components, which may cause coloration and putrefaction, are extracted and removed previously, so that there arises no adverse effect on the dispersibility of microcapsules nor possibility of putrefaction, which greatly contributes to stable production of microcapsules on a commercial scale.

Thus, according to the present invention, there is provided a means of microencapsulation using microorganisms which is excellent in industrial productivity and product quality.

### Detailed Description of Preferred Embodiments

The present invention relates to a process for the preparation of microcapsules which uses yeast cells as the microcapsule wall and which enables a hydrophobic liquid to be enclosed in a larger amount and more rapidly in the yeast cells. Basically the process comprises the following three steps.
1) The step of eluting the intracellular components to the outside of the yeast cell.
2) The step of mixing a previously prepared hydrophobic liquid with a suspension of the yeast cell residue to enclose the former in the latter.
3) The step of encapsulation associated with heating and stirring.

It is also possible to incorporate the step of dehydration or drying of the yeast cells or their residue into the process steps if necessary.

In the elution step of 1), those methods are basically preferred which can keep the deterioration and damage of the cell wall, which is to become the capsule film, to the minimum and attain a high elution rate. The "elution rate" referred to herein means the ratio of the absolute dry weight of the eluted components to the absolute dry weight of the initially added untreated yeast cells.

The elution of intracellular components to the outside of yeast cells may be accomplished by treating a yeast cell suspension at a suitable temperature and pH for a predetermined time, or treating the suspension with addition of an elution promoting agent, or treating it with addition of an enzyme preparation. The elution treatment is more effectively performed with simultaneous stirring. The treating time is not less than 1 hour, though it varies depending on the treating temperature. In the range of treating temperature of 30-60°C, it is not less than 1 hour, preferably not less than 5 hours. However, too long a treating time is unfavourable and the time should be kept within the range where no denaturation of the cell wall of the microorganism takes place. The above-mentioned treating temperature range is desirable in order to keep the deterioration and damage of the yeast cells to the minimum. To accomplish the elution of intracellular components more effectively and rapidly, an elution promoting agent is added. The elution promoting agents which may be used are, for example, polar organic solvents such as lower alcohols, ethyl acetate and acetone, inorganic salts, sugars, papain, quaternary ammonium salts, various germproof, anti-bacterial, and germicidal agents, etc. Particularly preferably used among them are ethanol and acetone.

A yeast cell suspension is usuably prepared by suspending yeast cells in water.

These elution promoting agents are added in an amount of less than 40 g, preferably in the range of 1.0 - 20 g, relative to 100 g of the yeast cell suspension.

The higher the elution rate of the intra-cellular components attainable by the above-mentioned method of elution, the larger the amount of the hydrophobic liquid which can be enclosed in the cells. A desirable elution rate is 30-70% by weight. Although a higher amount of the liquid can be enclosed by advancing the elution to a still higher range, it will cause even some components of the cell wall, which corresponds to the capsule wall, to be dissolved out by the elution, so that the wall will gradually lose its stoutness. Accordingly, it is desirable to avoid excessive elution.

After the elution treatment of intracellular components, the eluted components and the cell residue are preferably separated from each other by centrifugation or other means. That is, although the method disclosed in JP-A-No. 63-88,033 proposed previously by the present inventors also can attain a large amount of intake into the cell, the elution of intracellular components and the permeation of hydrophobic liquid proceed simultaneously in the method, so that the capsule suspension obtained is inevitably contaminated by the intracellular components and resultantly the suspension is susceptible to coloration, viscosity increase and putrefaction of the liquid and coagulation of capsule particles, which may cause trouble in the production step. However, such problems can be resolved when the eluted components are previously removed according to the process of the present invention.

The components which constitute the yeast cells are roughly classified as follows.
(1) Water-insoluble components based on glucan and mannan, which mainly constitute the cell wall.
(2) Phospholipid and other lipid components, which mainly constitute the cell membrane.
(3) Enzymes, proteins, amino acid components, saccharides, and nucleic acid components, which are soluble in water or polar solvents. The distribution of these components varies with specices of yeast. The intracellular components intended for as the target of elution in the present invention are mainly the components of (3), but the components of (2) may also become the target of elution depending on the kind of elution promoting agents, the kind of enzyme preparations, and treatment conditions.

The term "yeast(s)" referred to herein is a general term for microorganisms which multiply through budding or division.

Yeasts are in various shapes depending on the kinds of yeast, which include an oval, sphere, lemon shape, pillar and ellipse. Those having such shapes as a sphere, lemon and oval are preferred. They preferably have a particle diameter of 1-20 »m.

These yeasts may be used in the present invention either in the live state as such, or in the dead state, or also further in a dried state.

The hydrophobic liquids to be encapsulated in yeast cells are not particularly restricted so long as they are a liquid substantially insoluble in water or a substance which becomes such a liquid by heating. As specific examples thereof there may be mentioned those used for microcapsules made of synthetic resins, for example, oily liquid extracted from animals and plants, including cotton seed oil, soybean oil, corn oil, olive oil, castor oil, fish oils, various fatty acids and various steroids, and particularly when the microcapsules are used for carbonless copying paper, paraffin oil, chlorinated paraffin, chlorinated diphenyl, dibutyl phthalate, dioctyl phthalate, dibutyl maleate, o-dichlorobenzene, alkylated naphthalenes such as diisopropylnaphthalene, 1-phenyl-1-xylylethane, etc.

Into these hydrophobic liquids are dissolved or dispersed, according to necessity, dyes, perfumes, pharmacologically active substances, food materials, feed materials, etc. The microcapsules thus obtained may be used, besides for carbonless copying paper, for cosmetics, phamaceuticals, foods, feeds, agricultural chemicals, etc. The above-mentioned substances may be used also as such alone so long as they are hydrophobic liquids immiscible with water-soluble liquids.

The mixing of the hydrophobic liquid with the yeast cell suspension is usually performed by adding the hydrophobic liquid into the yeast cell suspension. In said mixing, although the hydrophobic liquid may be added as such alone into the yeast cell suspension, it is preferably added after dispersed in an aqueous solution containing a suitable emulsifier into the form of emulsion in order that it may be mingled in a more uniform condition with yeast cells.

The temperature in the encapsulation step is not particularly limited but is preferably 20-70°C. A time of 1 hour or more is necessary, which may be varied appropriately according to the amount of the hydrophobic liquid to be encapsulated and the encapsulation temperature.

At the encapsulation step there may be added according to necessity, besides the elution promoting agent, enzyme preparation and emulsifier mentioned above, also catalysts, film hardeners, pH regulating agents, antiseptics, and various degradation inhibitors.

The substance of the present invention differs distinctly from that disclosed by JP-A-No. 58-107,189 at the following points. First, the process of the present invention comprises the step of extracting as large an amount of intracellular components as possible from yeast cells prior to the step of encapsulating a hydrophobic liquid. Secondly, whereas the microorganisms used in JP-A-No. 58-107,189 are specified as having a lipid content of 10% by weight or more and the yeasts intended for as such microorganisms are those of a high lipid content, namely so-called lipid-accumulating yeasts, the yeasts used in the present invention have a low lipid content, and, preferably, the yeast have a low lipid content of less than 10% by weight (including 0% by weight) when the yeast cells are eluted using an elution promoting agent. These two kinds of yeasts exhibit an essential difference when they come into contact with a polar organic solvent, such as a lower alcohol, ethyl acetate and acetone, mentioned above as the specific examples of the elution promoting agent. That is, when a yeast of a high lipid content contacts with these polar solvents the solvents are taken into the cell as a "lipid- extending agent", whereas when the solvents come into contact with a yeast of a low lipid content intended for when using an elution promoting agent in the present invention, the solvents act as an "elution promoting agent" to elute the intracellular components and basically the solvents are not taken into the cell. Thus, the solvent exerts utterly different effects on respective kinds of yeast.

As set forth above, although the process of the present invention is in common with that disclosed by JP-A-No. 58-107,189 with respect to adding a polar organic solvent, the two processes are fundamentally different with respect to the presence or absence of the elution step, the composition of the yeast cells to be used, and the effect of the polar organic solvent exerted on the respective composition.

### (E) Example

The present invention will be described in detail below with reference to Examples, but it is in no way limited thereto.

The weights of yeast shown in the Examples and Comparative Examples all refer to the weights of live yeast at the dry state.

### Example 1

To 100 g of a suspension containing 10 g of a commercially available yeast (a live yeast, "Saccharomyces cerevisiae", manufactured by Kanegafuchi Chemical Industry, Co., Ltd., lipid content: 2% by weight) in water was added 10 g of ethanol and the resulting mixture was shaked in a rotary shaker at a temperature of 40°C for 24 hours to elute the water-soluble components in the cell to the outside of the cell. After separation of the eluate from the yeast cells residue by centrifugation, the whole eluate was exposed in a dryer at 105°C to evaporate off water. Resultantly 6.0 g of non-volatile components were left behind, confirming that 60% by weight of the initially added yeast cells had been eluted.

Then, 22 g of a high boiling temperature hydrophobic liquid (HISOL SAS N-296, a trade name, mfd. by Nippon Petrochemicals Co., Ltd.) containing 1.1 g of 3-N-methylcyclohexylamino-6-methyl-7-anilinofluoran (a black-color developing dye, trade name: PSD-150, mfd. by Nippon Soda Co., Ltd. was added as a hydrophobic liquid to 20 g of an aqueous solution containing 0.5% by weight of a nonionic surface active agent (Tween 80, a trade name, mfd. by Kao Atlas Co., Ltd.) as an emulsifier, with vigorous stirring, to obtain an emulsion of the hydrophobic liquid having an average particle diameter of 8 »m. The emulsion was added to the water suspension of the yeast cell residue obtained above and the resulting mixture was shaped in a rotary shaker at a temperature of 50°C and a stirring rate of 200 rpm for 3 hours. Resultantly, the hydrophobic liquid was wholly enclosed in the yeast cells and thus microencapsulation was completed. The microcapsule suspension obtained was bar-coated as such on fine paper of 40 g/m² at a coating weight of about 5 g/m² to obtain an upper sheet for carbonless copying paper with a good color-developing property.

### Example 2

To 100 g of the same yeast suspension as that used in Example 1 was added 5 g of acetone, and the mixture was shaped at 40°C for 24 hours to elute the water-soluble components in the cell to the outside. By determination of the elution rate in the same manner as in Example 1, it was confirmed that 45% by weight of the initial yeast cells had been eluted. Then microcapsules were prepared by using the yeast cell residue suspension obtained above in the same manner as in Example 1. The microcapsules thus prepared were coated on fine paper to obtain an upper sheet for carbonless copying paper with a good color-developing property.

### Example 3

To 15 g of a torula yeast (Candida utilis) obtained by aerobic cultivation was added 90 g of a 6% by weight sodium chloride solution, the resulting suspension was adjusted to pH 6.0 and then kept at 90°C for 4 hours to elute the intracellular components. Determination of the elution rate made in the same manner as in Example 1 confirmed that 30% by weight of the initial yeast cell had been eluted.

Then, microcapsules were prepared in the same manner as in Example 1 by using the yeast cell residue suspension obtained above. The microcapsules thus prepared were coated on fine paper to obtain an upper sheet for carbonless copying paper with a good color-developing property.

## Claims

1. A process for producing microcapsules from yeast cells comprising eluting intracellular components of yeast cells in suspension to outside of the yeast cells; and mixing the suspension of the yeast cells, from which intracellular components have been eluted, with a hydrophobic liquid thereby to obtain microcapsules containing the hydrophobic liquid; characterised in that the yeast cells have a low lipid-content of less than 10% by weight and are subjected to elution treatment at a temperature of 30 to 100°C for a period of time of not less than 1 hour in the absence or presence of an elution promoting agent, so that the intracellular components are eluted at a rate of 10 to 80% by weight and the yeast is a member selected from the group consisting of Saccharomyces cerevisiae, Saccharomyces rouxii, Saccharomyces carlsbergensis, Candida utilis, Candida tropicalis, Candida lipolytica and Candida flaveri.

2. A process according to claim 1, wherein the elution promoting agent is a polar organic solvent.

3. A process according to claim 1 or 2, wherein the elution promoting agent is a lower alcohol, ethyl acetate or acetone.

4. A process according to claim 3, wherein the elution promoting agent is ethanol.

5. A process according to claim 1, wherein the elution promoting agent is an inorganic salt.

## Patentansprüche

1. Verfahren zur Herstellung von Mikrokapseln aus Hefezellen umfassend
Eluieren intrazellulärer Komponenten von Hefezellen in Suspension aus den Hefezellen heraus; und Vermischen der Suspension der Hefezellen, aus denen intrazellulärer Komponenten eluiert wurden, mit einer hydrophoben Flüssigkeit, um dadurch Mikrokapseln zu erhalten, die die hydrophobe Flüssigkeit enthalten;
dadurch **gekennzeichnet,** daß die Hefezellen einen niedrigen Lipidgehalt von weniger als 10 Gew.% haben und bei einer Temperatur von 30 bis 100°C, für einen Zeitraum von nicht unter 1 Stunde, in Abwesenheit oder Anwesenheit eines Elutionspromotors einer Elutionsbehandlung unterzogen werden, so daß die intrazellulären Komponenten mit einer Rate von 10 bis 80 Gew.% eluiert werden, und daß die Hefe ein Glied, ausgewählt aus der aus Saccharomyces cerevisiae, Saccharomyces rouxii, Saccharomyces carlsbergensis, Candida utilis, Candida tropicalis, Candida lipolytica und Candida flaveri bestehenden Gruppe ist.

2. Verfahren nach Anspruch 1, in dem der Elutionspromotor ein polares organisches Lösungsmittel ist.

3. Verfahren nach Anspruch 1 oder 2, in dem der Elutionspromotor ein niedrigerer Alkohol, Ethylacetat oder Aceton ist.

4. Verfahren nach Anspruch 3, in dem der Elutionspromotor Ethanol ist.

5. Verfahren nach Anspruch 1, in dem der Elutionspromotor ein anorganisches Salz ist.

## Revendications

1. Procédé pour la production de microcapsules à partir de cellules de levure, comprenant les opérations consistant à éluer des composants intracellulaires des cellules de levure en suspension vers l'extérieur des cellules de levures ; et à mélanger la suspension des cellules de levure, à partir desquelles les composants intracellulaires ont été élués, avec un liquide hydrophobe permettant ainsi d'obtenir des microcapsules contenant le liquide hydrophobe ; caractérisé en ce que les cellules de levure ont une faible teneur en lipides, inférieure à 10 % en poids et sont soumises au traitement d'élution à une température de 30 à 100°C pendant une durée non inférieure à une heure à l'absence ou en présence d'un agent facilitant l'élution de façon à éluer les composants intracellulaires à raison de 10 à 80 % en poids et la levure est un élément choisi dans le groupe constitué par les Saccharomyces cerevisiae, Saccharomyces rouxii, Saccharomyces carlsbergensis, Candida utilis, Candida tropicalis, Candida lipolytica et Candida flaveri.

2. Procédé selon la revendication 1, dans lequel l'agent favorisant l'élution est un solvant organique polaire.

3. Procédé selon la revendication 1 ou 2, dans lequel l'agent facilitant l'élution est un alcool inférieur, un acétate d'éthyle ou l'acétone.

4. Procédé selon la revendication 3, dans lequel l'agent facilitant l'élution est l'éthanol.

5. Procédé selon la revendication 1, dans lequel l'agent facilitant l'élution est un sel minéral.
